# EUROPÄISCHE PATENTSCHRIFT

(11) **EP 0 890 358 B1**
(45) Veröffentlichungstag und Bekanntmachung des Hinweises auf die Patenterteilung: **09.02.2005**
(21) Anmeldenummer: 97111783.3
(22) Anmeldetag: 10.07.1997
(51) Int. Cl.: A61K 9/20, A23G 3/02

(54) **Lösliche, gummihaltige, dragierte Kautablette**
Soluble, dragée-made chewable tablette containing gum
Comprimé à macher soluble, dragéifié, contenant des gommes

(43) Veröffentlichungstag der Anmeldung: 13.01.1999
(73) Patentinhaber: Dr. Gergely & Co., 1053 Wien (AT)
(72) Erfinder: Gergely, Gerhard, Dr., 1053 Wien (AT); Gergely, Irmgard, 1053 Wien (AT); Gergely, Thomas, Dr., 1053 Wien (AT)
(74) Vertreter: Bogensberger, Burkhard, Dr.

(56) Entgegenhaltungen:
- EP-A- 0 212 641
- WO-A-86/03967
- GB-A- 1 538 280
- DATABASE WPI Section Ch, Week 9610 Derwent Publications Ltd., London, GB; Class B07, AN 96-088696 XP002049401 & DK 9 301 073 A (JORGENSEN APS H B) , 24.März 1995

## Beschreibung

Kaugummizubereitungen auf Basis von Naturkautschuk sind in der Pharmaindustrie vielfach verwendet worden. Ihre Vorteile sind die angenehme und beliebte Darreichungsform, sowie eine rasche, sublinguale Resorption eines Wirkstoffes. Der schwerwiegende Nachteil solcher Zubereitungen ist aber die nach dem Kauen übrigbleibende Gummimasse, die dazu geführt hat, dass z. B. in Flugzeugen Antiemetika auf dieser Basis unerwünscht sind und beispielsweise in Singapur der Besitz von Kaugummis bereits strafbar ist. So beschreibt die WO86/03967A eine Tablette in der die Kaugummibase neben Füll-, Zuschlag- und Wirkstoffen in einer Matrix aus Fetten und Wachsen vorliegt.

Andererseits ist es auch aus der Zuckerwarenindustrie bekannt, Gummi arabicum und zuckerhältige Lösungen zu kochen und in geeignete Formen zu vergiessen, die anschliessend gegebenenfalls dragiert werden. Zum Beispiel beschreiben die beiden US Patente 4,698,232 und 5,476,678 faserhältige kaubare Massen mit einer geschäumten Matrix aus Gummi arabicum und Gelatine, sowie einer nicht geschäumten Matrix aus Zuckersirup.

Die EP-A-212641 beschreibt eine Arzneimittel-Polymermatrix aus Methacrylsäure und deren Methylester zur Geschmacksmaskierung, die in einem Medium mit einem pH-Wert kleiner als 4 (Magensaft) dissoziert und dabei den Wirkstoff in das Medium freigibt. Die DE-A1-4444051 beschreibt eine Kautablette, die im Mund rasch zerfällt, und bei der hydrophile Wirkstoffteile hydrophob beschichtet oder eingebettet sind, gegebenenfalls mit bzw. in Gummi arabicum. Die GB-B-1142377 beschreibt eine Kautablette für die Zahnreinigung, bei der je ein Teil Gummi arabicum und Gelatine mit zwei Teilen Glycerin und verschiedenen kleinen Zuschlagstoffmengen gemischt werden. Die GB-A-1538280 beschreibt eine Tablette auf Zuckerbasis mit hohem Fettanteil und dadurch verminderter Härte, die deshalb beim Kauen leicht aufbricht und somit den Wirkstoff schnell und vollständig abgibt. Die Fettphase bildet darauf in der Speiseröhre einen Überzug und schützt weiters den Wirkstoff vor dem Angriff durch die Magensäfte.

Für die Herstellung einer pharmazeutischen Zubereitung sind alle diese Produkte bzw. deren Herstellverfahren nicht oder nur beschränkt geeignet. Einerseits will man für verschiedene Wirkstoffe, z. B. Antiemetika oder Desinfizientien, eine langsam, über mehrere Minuten verteilte Abgabe an den Körper erzielen; die bisher für diesen Zweck ideale Zubereitung in einem Kaugummi stösst - wie bereits erwähnt - in zunehmendem Masse auf Widerstand. Andererseits ist das aus der Zuckerwarenindustrie bekannte Herstellverfahren für die hohen Anforderungen einer Good Manufacturing Practice in der pharmazeutischen Industrie oft nicht anwendbar und für kleinere Chargenmengen auch vom apparativen Aufwand her viel zu teuer.

Die Erfindung hat sich daher die Aufgabe gestellt, eine Zusammensetzung und ein Verfahrenzu schaffen, bei dem Wirkstoffe, wie Hustenlöser (Clobutinol, Salbutamol), Rachendesinfizientien, Vitamine und/oder Spurenelemente auch in geringer Dosierung in eine lösliche Kaugummimasse entsprechend dosisgenau eingearbeitet werden können, die mit einer den pharmazeutischen Anforderungen entsprechenden Technologie zu Tabletten verpresst werden können, die eine lösliche, kaubare Masse ergeben.

Diese Aufgabe wird erstmals in überraschender Weise durch die Kombination der in Anspruch 1 festgelegten Merkmale bzw. Massnahmen gelöst, und zwar dadurch, dass infolge der Zusammensetzung und des Verfahrens eine körnige gummihaltige Masse entsteht, die zu einer löslichen, gummihaltigen, dragierten Kautablette weiterverarbeitet wird. Weitere und bevorzugte Ausbildungen der Erfindung sind in den Merkmalen der abhängigen Ansprüche beschrieben. Erfindungsgemäss wird dieses neue Produkt und Herstellverfahren vorgeschlagen, das die obgenannten Nachteile vermeidet bzw. die gewünschten Anforderungen erfüllt. Dies gelingt dadurch, dass die Zusammensetzung und das Verfahren eine körnige Masse bietet, wobei durch Kühlung eine gute Verarbeitbarkeit - insbesondere beim Zerkleinern und Zumischen weiterer Zuschlagsstoffe, sowie hinsichtlich der Verpressbarkeit - gegeben ist.

Im Sinne der Erfindung sind unter Kaukomponenten" alle solchen Substanzen zu verstehen, die allein oder in Mischung miteinander beim Kauen wenigstens 1, vorzugsweise wenigstens 2, insbesondere wenigstens 3 min lang sich kaugummiartig verhalten, sich während dieser Zeit aber aufzulösen beginnen und danach völlig gelöst und mit dem Speichel geschluckt werden. In diesen Substanzbereich gehören unter anderem, aber nicht ausschliesslich: Gummi arabicum, Tragacanth, Guargum, Xanthangummi, Pektine; aber auch Trockensirupe, wie z.B. Trocken-Glucosesirup und/oder Fructosesirup; lösliche Cellulosederivate, wie z.B. Natriumcarboxymethylcellulose. Trockenglucosesirup zeigt ebenfalls beim Kauen teilweise gummiähnliches Verhalten.

Unter Sirupkomponenten" im Sinne der Erfindung werden demgegenüber ausschliesslich solche verstanden, die entweder als Sirup oder in hochkonzentrierter Lösung zum Einsatz kommen, wie z.B. 80%iger Glucosesirup, 70%ige Maltodextrinlösung, gequollene Gelatine, aber auch andere Sirupe, wie Mais-, Zuckeroder Invertzuckersirup (Invertin® Merck). Es kann Glucosesirup ganz oder teilweise durch andere konzentrierte Kohlehydrat-, Zuckeralkohol-, Gelatine- oder dgl. -Lösungen ersetzt werden. Wichtig ist die Einhaltung einer möglichst geringen Wassermenge, da die Mischung ansonsten breiförmig wie im Stand der Technik wird. Andererseits muss eine bestimmte Feuchtigkeitsmenge von etwa 4 bis etwa 7 % im Dragee verbleiben, da die Tablette beim Austrocknen und bei einem Feuchtigkeitsgehalt von unter 2% keine geschmeidige, kaufähige Masse mehr ergeben würde und der Anbiss zu hart wäre.

Als Fettkomponenten können alle essbaren, tierischen und pflanzlichen Fette eingesetzt werden. Es sind dies Triglyceride, die im wesentlichen aus Gemischen von Glycerinestern höherer Fettsäuren, insbesondere pflanzlichen oder tierischen Ursprungs, etwa der Grössenordnung C₁₀ bis C₂₂, bestehen, deren Schmelzpunkt nicht über 60°C liegt.

Besonders vorteilhaft hinsichtlich der Kaubarkeit hat sich der Zusatz von Wachsen, wie z. B. Bienenwachs, festem Paraffin, Ozokerit oder ähnlichen Substanzen erwiesen, die eine längere Kaudauer gewährleisten. Insbesondere der Zusatz von Bienenwachs verbessert die Kaufähigkeit und reduziert das Ankleben an den Zähnen. Der Zusatz relativ geringer Mengen an Glycerin oder Propylenglykol macht den Anbiss des Drageekerns weicher.

Die Verarbeitung erfolgt grundsätzlich nach folgendem Schema: Alle Pulverbestandteile werden in einen Flächenmischer eingebracht und auf langsamer Stufe gemischt. Anschliessend werden Glycerin und eine Schmelze aus Fetten und Wachsen eingebracht und sorgfältig mit dem Pulver gemischt. Schliesslich fügt man einen - z.B. kohlehydrathältigen - Sirup hinzu, der bei Bedarf - z. B. zur Reduktion der Viskosität zwecks Erleichterung der Verarbeitung - auf ca. 40°C erwärmt werden kann, wobei aber auch nicht erwärmter Glucosesirup Verwendung finden kann. Man mischt so lange, bis eine krümelige Masse entsteht. Der Zusatz des pharmazeutischen Wirkstoffes erfolgt je nach seiner Art, d.h. unter anderem nach seinem Geschmack und/oder seinen Stabilitätskriterien; ist er stabil und geschmacksneutral, dann einfach durch Zumischung als Pulver in die Ausgangsmischung; in einem anderen Fall beispielsweise durch Auflösung in der Fettschmelze, eingehüllt in ein Hydrokolloid, oder in einer durch geeignete Massnahmen geschmacksmaskierten Matrix, z.B. im Falle von Dimenhydrinat in Polymethacrylsäureestern, HPMCP (Hydroxypropylmethylcellulosephtalat), Alginsäure u.dgl.

Das Kühlen der krümeligen Masse kann entweder batchweise in Säcken aus Kunststoff-Folie, z.B. in einem Kühl- bzw. Tiefkühlschrank, oder auch im Durchlauf auf einem Kühl-Förderband erfolgen. Z.B. werden die Säcke in ein Bett von Kohlensäureschnee gelegt und mit Kohlensäureschnee abgedeckt. Im Anschluss daran kann die Masse - z.B. durch Mahlung und Siebung - auf die gewünschte Korngrösse von etwa 2 bis etwa 5 mm zerkleinert werden. Anschliessend werden zu dieser zerkleinerten Masse die Zusatzstoffe zugemischt, wie z.B. zusätzliche künstliche Süssstoffe, Aromen u. dgl., sowie Wirkstoffe bzw. Wirkstoffgemische, eine Wirkstoffmatrix etc. Die Endmischung wird dann - zweckmässigerweise über eine Kühlstrecke, z.B. durch einen Doppelmantel, der aussen mit Kühlsole gekühlt ist - zu einer Tablettenpresse, vorzugsweise einem Rundläufer, geführt und zu Tabletten gewünschter Grösse verpresst.

Die Temperatur, auf die tiefgefroren bzw. gekühlt wird, ist auf die jeweilige Mischung abzustimmen. Die einzelnen Mischungsbestandteile bilden miteinander ein komplexes System und beeinflussen einander gegenseitig im Hinblick auf das Verhalten während des Mahlvorganges bzw. der Verpressbarkeit. Ist der Feuchtegehalt an der oberen Grenze, muss auf tiefere Temperaturen gefroren werden. Ferner ist darauf zu achten, dass die Verarbeitung möglichst in einem geschlossenen System und/oder in einem Raum mit geringer Luftfeuchtigkeit und/oder während kurzer Zeiträume erfolgt, um die Kondensation von Luftfeuchtigkeit auf der Masse so gering wie möglich zu halten, bzw. diese Feuchtigkeit bei der Rezepterstellung zu berücksichtigen.

Die Tabletten können anschliessend als Drageekerne in an sich bekannter Weise dragiert werden. Beim Dragieren werden die Kerne in der Regel auf etwa 40 bis etwa 50°C angewärmt. Bei dieser Temperatur durchdringt die Feuchtigkeit, die mit der konzentrierten Sirup- und/oder Gelatinelösung zugeführt wurde, die Kaukomponenten, und es entsteht eine sehr gut kaubare Masse, wobei der Trockensirup die Kaubarkeit begünstigt bzw. verbessert.

Das im Anspruch 1 definierte Produkt hat - vom Verpressen der gekühlten Granulatteilchen her - vermutlich eine teilkörnige Struktur, in der durch die tiefe Temperatur die Feuchtigkeit zunächst immobilisiert wird. Nach dem Verpressen wird diese Feuchtigkeit durch Erwärmung (insbesondere während des Dragiervorganges) beweglich und wandert bzw. diffundiert - zunächst oberflächlich - in die vorhandenen, wasserlöslichen Inhaltsstoffe. Diese werden angelöst und ergeben eine hochviskose, thixotrope, kaufähige Masse.

Die Erfindung wird im folgenden anhand der Beispiele näher erläutert.

### Beispiele 1 bis 8:

Zunächst wird die Variabilität der einzelnen Komponenten gezeigt. Die verschiedenen Zusammensetzungen sind in Tabelle 1 einander gegenübergestellt.

In den Beispielen 1 bis 3 werden die Wachse variiert: 1. Paraffin, 2. Ozokerit, 3. Bienenwachs.

In den Beispielen 2 bis 4 werden die Fett- bzw.Wachskomponenten variiert: Kokosfett, pflanzliche und tierische Triglyceride.

In den Beispielen 5 bis 8 werden verschiedene Kaukomponenten und - damit im Zusammenhang - auch verschiedene kohlehydrathältige Sirupe, sowie gequollene Gelatine, bzw. im Beispiel 7 ein Zuckeralkohol eingesetzt.

Die Herstellung der erfindungsgemässen Mischungen in diesen Beispielen geschieht wie folgt:
Für die Beispiele 1 bis 4 werden das sprühgetrocknete Gummi arabicum, Reisstärke, Trockenglucosesirup und Aspartam in einen Flächenmischer eingebracht und auf langsamer Stufe gemischt. Anschliessend wird Glycerin eingebracht und verteilt, und schliesslich wird die auf etwa 40°C geschmolzene Mischung des Kokosfettes mit Paraffin bzw. Ozokerit (Beispiele 1 und 2), bzw. die Schmelze aus Margarine oder tierischem Fett, jeweils mit Bienenwachs (Beispiele 3 und 4), aufgebracht und eingemischt. Schliesslich wird der flüssige Glucosesirup eingerührt. Nach 5 min sorgfältigem Mischen wird die Masse auf etwa -12°C gefroren, anschliessend auf 2,0 bis 3,5 mm gemahlen, gekühlt und schliesslich zu Tabletten verpresst, die sodann dragiert werden.

Für die Beispiele 5 und 6 arbeitet man analog, nur wird statt sprühgetrocknetem Gummi arabicum Tragacanth eingesetzt (Beispiel 5), und in Beispiel 6 wird statt des 80%igen Glucosesirups eine 70%ige Maltodextrinlösung und dazu Maltodextrinpulver verwendet. Für die Beispiele 7 und 8 wird zunächst die Gelatine in der Zitronensäure-Wasser-Lösung quellen gelassen; anschliessend rührt man Mannit bzw. Lactose ein. Die erhaltene Masse wird in einen Mischkessel gebracht, in dem sprühgetrocknetes Gummi arabicum, Reisstärke und Aspartam bereits vorgemischt vorliegen. Anschliessend werden in analoger Weise Glycerin, die Kokosfett-Bienenwachsschmelze und der Glucosesirup eingebracht und verteilt. Die Masse wird anschliessend gefroren, gemahlen, neuerlich gekühlt und verpresst.

**Tabelle 1**

| Bespiel Nr. | 1 | 2 | 3 | 4 | 5 | 6 | 7 | 8 |
|---|---|---|---|---|---|---|---|---|
| Gummi arabicum | 18,14 | 18,14 | 18,14 | 18,14 | | 18,14 | 18,14 | 18,14 |
| Glycerin | 0,31 | 0,31 | 0,31 | 0,31 | 0,31 | 0,31 | 0,31 | 0,31 |
| Reisstärke | 8,32 | 8,32 | 8,32 | 8,32 | 8,32 | 8,32 | 8,32 | 8,32 |
| Glucosesirup (getrocknet) | 27,20 | 27,20 | 27,20 | 27,20 | 27,20 | 27,20 | | |
| Bienenwachs | | | 0,67 | 0,67 | 0,67 | 0,67 | 0,67 | 0,67 |
| Kokosfett | 6,13 | 6,13 | | | 6,13 | 6,13 | 6,13 | 6,13 |
| Glucosesirup (flüssig) | 39,04 | 39,04 | 39,04 | 39,04 | 39,04 | | 39,04 | 39,04 |
| Aspartam | 0,19 | 0,19 | 0,19 | 0,19 | 0,19 | 0,19 | 0,19 | 0,19 |
| Paraffin | 0,67 | | | | | | | |
| Ozokerit | | 0,67 | | | | | | |
| Margarine | | | 6,13 | | | | | |
| tierisches Fett | | | | 6,13 | | | | |
| Tragacanth | | | | | 18,14 | | | |
| Maltodextrin (Pulver) | | | | | | 18,85 | | |
| Maltodextrin (70%ige Lsg.) | | | | | | 20,19 | | |
| Mannit | | | | | | | 25,07 | |
| Zitronensäure | | | | | | | 0,11 | 0,20 |
| Wasser | | | | | | | 0,86 | 1,70 |
| Gelatine | | | | | | | 1,16 | 2,27 |
| Lactose | | | | | | | | 23,03 |
| | 100,00 | 100,00 | 100,00 | 100,00 | 100,00 | 100,00 | 100,00 | 100,00 |

**Tabelle 2**

| Bespiel Nr. | Kaubarkeit | Kaudauer |
|---|---|---|
| 1 | nicht bröselig, kaubar, geschmeidig | 110 - 120 sec |
| 2 | nicht bröselig, kaubar, geschmeidig | 105 - 125 sec |
| 3 | nicht bröselig, kaubar, geschmeidig | 110 120 sec |
| 4 | nicht bröselig, kaubar, geschmeidig | 95 - 100 sec |
| 5 | nicht bröselig, gut kaubar, | 100 -110 sec |
| 6 | nicht bröselig, gut kaubar, geschmacklich gut | 105 - 115 sec |
| 7 | nicht bröselig, gut kaubar, geschmeidig | 35 - 40 sec |
| 8 | nicht bröselig, angenehmes Zerfallsverhalten | 30 - 35 sec |

Die Ergebnisse der Beispiele 1 bis 8 sind in Tabelle 2 dargestellt, wobei sich zeigt, dass bei Verwendung von Mannit und Lactose die Kaudauer verkürzt ist.

### Beispiele 9 bis 12

Diese Beispiele zeigen verschiedene Varianten möglicher Kombinationen und deren Mengen von Kaukomponenten, von Fetten und Wachsen, sowie von kohlehydrathältigen Sirupen.

Die Zusammensetzungen finden sich in Tabelle 3. Die Herstellung erfolgt analog wie bei den vorgenannten Beispielen. Die Ergebnisse finden sich in Tabelle 4. Für Beispiel 12 soll das Herstellverfahren im folgenden nochmals detailliert festgehalten werden (alle Mengen in Gewichtsprozent):

18,1% sprühgetrocknetes Gummi arabicum, 27,1% Trocken-Glucosesirup, 8,3% Reisstärke und 0,3% Aspartam werden in einem Flächenmischer 5 min lang gemischt, sodann mit 0,3% Glycerin versetzt und bei mittlerer Rührgeschwindigkeit 1 bis 2 min lang gemischt. Anschliessend bringt man eine auf 40°C erwärmte, flüssige Fettschmelze aus 6,1% gehärtetem Kokosfett, 0,7% Bienenwachs und 0,2% Pfefferminzöl ein und verteilt 3 min lang in der Mischung. Danach wird unter ständigem Rühren 39,0% 80%iger Glucosesirup eingebracht und 5 min lang gleichmässig verteilt. Die resultierende, krümelige Masse wird gefroren und in einer Reibschnitzelmühle auf 3,5 mm Korngrösse vermahlen. Das erzielte Granulat wird nochmals auf 0 bis -10°C gekühlt und bei 0°C zu Tabletten verpresst, die anschliessend dragiert werden.

**Tabelle 3**

| Beispiel Nr. | 9 | 10 | 11 | 12 |
|---|---|---|---|---|
| Gummi arabicum (Spraygum) | 55,20 | 43,19 | 29,13 | 18,15 |
| Reisstärke | | | 3,78 | 8,33 |
| Glucosesirup getrocknet | | | 29,13 | 27,20 |
| Bienenwachs | | | | 0,67 |
| Kokosfett gehärtet | 5,53 | 4,29 | 2,91 | 6,13 |
| Glucosesirup flüssig | 38,68 | 42,92 | 34,65 | 39,00 |
| Pfefferminzöl | 0,19 | 0,19 | 0,11 | 0,19 |
| Aspartam | 0,40 | 0,33 | 0,29 | 0,33 |
| Zitronensäure Pulver | | 0,43 | | |
| Gelatine | | 8,65 | | |
| | 100,00 | 100,00 | 100,00 | 100,00 |

**Tabelle 4**

| Beispiel Nr. | Kaubarkeit | Kaudauer | Preßtemperatur |
|---|---|---|---|
| 9 | nicht bröselig, angenehmes Zerfallsverhalten | 80˝ -85˝ | minus 15°C |
| 10 | nicht bröselig, angenehmes Zerfallsverhalten | 100˝ - 110˝ | minus 11°C |
| 11 | nicht bröselig, angenehmes Zerfallsverhalten | 100˝ - 110˝ | minus 8°C |
| 12 | nicht bröselig, angenehmes Zerfallsverhalten | 120˝ - 130˝ | minus 5°C |

### Beispiele 13 bis 16 (Negativbeispiele, siehe Tabelle 5)

Die Zusammensetzung nach Beispiel 13 wird so hergestellt, dass man zunächst Zucker und Zitronensäure in Wasser löst, Gelatine dazugibt und quellen lässt, und anschliessend Invertin zufügt. Man bringt dann das Gummi arabicum ein, schmilzt das Fett auf und arbeitet es ein. Anschliessend werden zuerst Tylose und Carboxymethylcellulose, sowie dann das Cyclodextrin zugegeben. Dann wird Ozokerit, Bienenwachs und Paraffin aufgeschmolzen und mit der vorher erzielten Masse vermengt. Man lässt dann tieffrieren, vermahlt und verpresst.

Das Produkt lässt sich aufgrund sehr starker Klebeeigenschaft schlecht verpressen, was einerseits auf die fehlende Fett- bzw.Wachskomponente, wie Kokosfett, andererseits auf die Zuckerlösung und Carboxymethylcellulose zurückzuführen sein dürfte, die relativ viel Feuchtigkeit aufnimmt und daher beim Pressdruck stärker klebt, sodass quasi kein Wasserfilm, bzw. Fettfilm entstehen kann, der beim Pressen als Gleitmittel dient.

Bei Beispiel 14 wird wie bei Beispiel 13 verfahren, nur statt Paraffin Witepsol® zugefügt. Die Masse lässt sich zwar verpressen, doch hat das Endprodukt eine relativ kurze Kaudauer und zerfällt zu schnell, was auf Witepsol® zurückzuführen sein dürfte, das im Mund aufgrund seines Schmelzpunktes (32°C) sehr schnell wegschmilzt und dadurch verhindert, dass sich beim Kauen eine zusammenkaubare Masse ergibt.

**Tabelle 5**

| Bespiel Nr. | 13 | 14 | 15 | 16 |
|---|---|---|---|---|
| Zucker | 24,34 | 19,15 | 28,85 | |
| Wasser | 4,80 | 3,74 | 7,27 | |
| Invertin | 0,36 | 0,28 | | |
| Gelatine | 3,54 | 2,77 | 6,61 | |
| Galafett 36 | 0,72 | 0,58 | | |
| Gummi arabicum | 8,00 | 6,28 | 9,61 | 79,92 |
| Tylose C 10 000 P | 1,44 | 1,13 | | |
| Cyclodextrin | 21,57 | 16,92 | | |
| Zitronensäure | 0,01 | 0,01 | 0,58 | |
| Paraffin | 7,69 | | | |
| Ozokerit | 13,07 | 20,55 | | |
| Bienenwachs | 8,46 | 13,23 | | |
| Caboxymethylcellulose 7HXF | 5,00 | | | |
| Pfefferminzaroma | 1,00 | | 1,00 | |
| Witepsol H32 | | 15,38 | | |
| Kokosfett gehärtet | | | 6,00 | 7,69 |
| Maisstärke | | | 3,50 | |
| Maltodextrin | | | 28,85 | |
| Glucosesirup flüssig | | | 7,23 | 15,39 |
| Aerosil | | | 0,50 | |
| | 100,00 | 100,00 | 100,00 | 100,00 |
| | | | | |
| Bemerkung: | läßt sich nicht pressen | zerfällt zu schnell beim Kauen | bröselt | |

Die Mischung nach Beispiel 15 wird so hergestellt, dass zunächst die Gelatine quellen gelassen und anschliessend mit dem Glucosesirup vermengt wird. In einem Flächenmischer werden die Feststoffe gemischt; es wird dann das geschmolzene Kokosfett daruntergemischt und schliesslich die Gelatine-Glucosesirup-Masse zugegeben. Die ganze Masse wird nun auf 80°C erwärmt, wobei sie teigig wird, anschliessend gefroren, mit Aerosil vermischt und vermahlen. Man mischt dann Aroma zu und kühlt die Masse vor dem Verpressen nochmals ab. Sodann fügt man 20 Teile Glucosesirup hinzu, den man unter Rühren gut verteiilen lässt.

Die Masse bleibt bröselig, was auf die Verdunstung des Wassers durch die Erwärmung auf 80°C zurückzuführen sein dürfte. Ausserdem wird durch diese Erwärmung die Migration von Feuchtigkeit in die Feststoffe gefördert, die in den Pulverteilchen festgehalten wird, bevorzugt in Substanzen wie Maisstärke und Maltodextrin, die z.B. 5 bis 7% Wasser zusätzlich zu ihrem eigenen Ausgangs-Wassergehalt von 5 bis 7% aufnehmen können, ohne ihre pulvrige Struktur zu verlieren. Diese Feuchtigkeit steht dann nach dem Verpressen beim Anwärmen, wie z.B. beim Dragieren, für die Erzielung des gewünschten Effektes einer kaubaren Masse nicht mehr zur Verfügung.

Für das Beispiel 16 werden 100 Teile sprühgetrocknetes Gummi arabicum bei Zimmertemperatur mit 10 Teilen geschmolzenem, gehärtetem Kokosfett versetzt und gut durchgerührt. Sodann fügt man 20 Teile Glucosesirup hinzu, den man unter Rühren gut verteilen lässt. Infolge des allzu hohen Anteils an Gummi arabicum ergibt sich ein zu starker Klebeeffekt an den Zähnen, eine zu geringe Kaudauer sowie ein etwas zu pappiges Gefühl beim Kauen im Munde.

### Beispiele 17 bis 23 (mit Wirkstoffen)

Es können verschiedenste Wirkstoffgruppen eingesetzt werden, wobei eine besondere Eignung für bucal resorbierende Wirkstoffe und für die Wirkstoffe, die im Mundund Rachenraum wirksam werden, wie Mund- und Rachendesinfizientia gegeben ist. Weiters können Vitamine und Mineralstoffe, husten- und schleimlösende Substanzen, auch Antiemetika und Antiallergika, in dieser Form verabreicht werden. Die Zusammensetzungen finden sich in Tabelle 6, die Herstellung erfolgt analog zu den Beispielen 1 bis 12, die Ergebnisse werden in Tabelle 7 wiedergegeben.

In den Beispielen 17 bis 20 wird als Wirkstoff Dimenhydrinat in einer geschmacksmaskierten Matrix, in den Beispielen 21 bis 23 ein handelsübliches, 50%iges Vitamin E-Pulver eingesetzt.

Die Wirkstoffmatrix für Beispiel 17 wird so hergestellt, dass man 60 Gewichtsteile Dimenhydrinat in 40 Gewichtsteilen Ethanol löst, dann 4 Gewichtsteile Natriumhydrogencarbonat in der Wirkstofflösung suspendiert und eine Lösung von 36 Gewichtsteilen Eudragit S 100 in 144 Gewichtsteilen Ethanol zufügt. Nach dem Vermischen wird das Lösungsmittel abgedampft, der Rückstand wird vakuumgetrocknet und auf 0,8 mm gesiebt.

**Tabelle 6**

| Beispiel Nr. | 17 | 18 | 19 | 20 | 21 | 22 | 23 |
|---|---|---|---|---|---|---|---|
| Gummi arabicum | 18,11 | 17,86 | 42,27 | 27,13 | 12,01 | 12,01 | 12,01 |
| Glycerin | 0,31 | 0,31 | | | 0,27 | 0,27 | 0,27 |
| Reisstärke | 2,76 | 2,09 | | 3,48 | 5,51 | 5,51 | 5,51 |
| Glucosesirup getr. | 27,09 | 26,67 | | 27,13 | 17,97 | 17,91 | 17,97 |
| Bienenwachs | 0,67 | 0,67 | | | 0,59 | 1,67 | 2,75 |
| Kokosfett gehärtet | 6,11 | 6,00 | 4,23 | 4,07 | 5,41 | 4,32 | 3,24 |
| Glucosesirup flüssig | 38,88 | 38,33 | 42,27 | 33,33 | 34,38 | 34,38 | 34,38 |
| Pfefferminzöl | 0,19 | 0,19 | 0,19 | 0,19 | | | |
| Aspartam | 0,33 | 0,33 | 0,33 | 0,33 | 0,29 | 0,29 | 0,29 |
| Calciumcarbonat | | 1,33 | | | | | |
| Wirkstoffmatrix | 5,55 | 6,22 | 4,00 | 4,33 | 23,58 | 23,58 | 23,58 |
| Zitronensäure Pulver | | | 0,42 | | | | |
| Gelatine | | | 6,29 | | | | |
| | 100,00 | 100,00 | 100,00 | 100,00 | 100,00 | 100,00 | 100,00 |

**Tabelle 7**

| Beispiel Nr. | Kaubarkeit | Kaudauer |
|---|---|---|
| 17 | nicht bröselig, angenehmes Kauverhalten | 90 - 100 sec |
| 18 | guter Geschmack | |
| | gut kaubar, geschmeidig | 110 - 120 sec |
| 19 | geschmeidig | 100 - 110 sec |
| 20 | angenehmes Zerfallsverhalten | 95 - 105 sec |
| 21 | gutes Kauverhalten, geschmeidig | 110 - 120 sec |
| 22 | gut kaubar | 90 -100 sec |
| 23 | gut kaubar, angenehmes Zerfallsverhalten | 110 - 120 sec |

Für Beispiel 18 wird in analoger Weise verfahren, nur werden 53,6 Gewichtsteile Dimenhydrinat in 40,2 Gewichtsteilen Ethanol gelöst; in der Lösung werden anschliessend 3,5 Gewichtsteile Natriumhdrogencarbonat und 10,7 Gewichtsteile Calciumcarbonat suspendiert. Danach mischt man eine Lösung von 32,2 Gewichtsteilen Eudragit S 100 in 128,7 Gewichtsteilen Ethanol zu und verfährt ansonsten wie bei Beispiel 17.

Für Beispiel 19 wurde nach Zusatz von Zitronensäure die Gelatine in dem Glucosesirup in der Wärme eine Stunde lang quellen gelassen und nicht gelöst, um nicht zu viel Wasser einzubringen. Diese Mischung wird in das mit geschmolzenem Kokosfett versetzte Gummi arabicum eingerührt und analog zu den vorangegangenen Beispielen gekühlt, gemahlen, neuerlich gekühlt und schlussendlich verpresst.

### Beispiele 24 bis 29 (Zusammensetzungen siehe Tabelle 8)

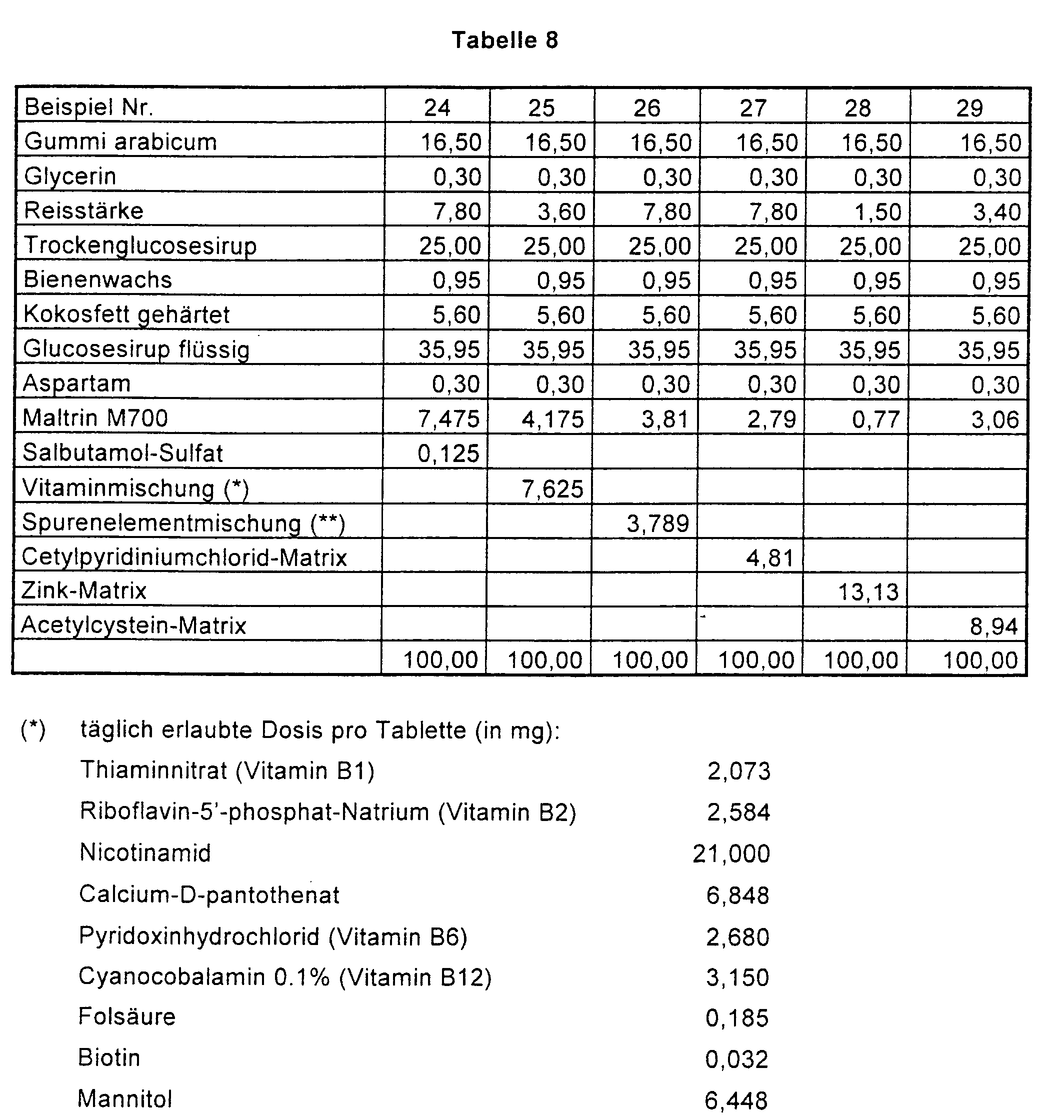

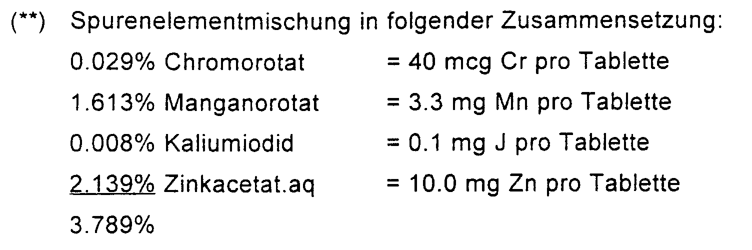

In diesen Beispielen wird eine konstante Zusammensetzung mit verschiedenen Wirkstoffen versetzt. Die analog zu den Beispielen 17 bis 23 mit sprühgetrocknetem Gummi arabicum (Spraygum) hergestellten Dragees bieten alle ein gutes Kauverhalten und angenehmen Geschmack. Es kann auch ein lösliches Kaudragee mit Multivitaminen hergestellt werden, wobei die Vitamine bevorzugt in der entsprechenden daily allowance"-Dosierung eingesetzt werden (siehe Beispiel 25).

Die Wirkstoffe der Beispiele 27 bis 29 werden in Form einer an sich bekannten geschmacksmaskierenden Matrix eingebracht. Die Dosierung entspricht dabei in Beispiel 27: 2 mg pro Tablette Cetylpyridiniumchlorid; in Beispiel 28: 25 mg Zink pro Tablette; und in Beispiel 29: 100 mg Acetylcystein pro Tablette.

Folgende Wirkstoffe stellen weitere Beispiele für die zweckmässige Anwendung der Erfindung dar: Herz-Kreislaufmittel, wie z.B.Salbutamol; Schleim- und Hustenlöser, wobei das Antitussivum Clobutinol augrund des bitteren Geschmacks in eine Matrix eingearbeitet werden muss; Antihistaminika wie Cetirizin, Loratadin; Mund- und Rachentherapeutika, wie Cetylpyridiniumchlorid, Benzalkoniumchlorid, Dequaliniumchlorid, die bevorzugterweise ebenfalls in Form einer Wirkstoffmatrix vorliegen, wobei auch eine Fett- und Wachs-Matrix hergestellt werden kann.

## Patentansprüche

1. Lösliche, gummihaltige, dragierte Kautablette, enthaltend 20 bis 50 Gewichtsprozent zumindest einer Sirupkomponente; 10 bis 60 Gewichtsprozent Kaukomponenten, die sich beim Kauen wenigstens 1 Minute lang kaugummiartig verhalten, sich während dieser Zeit aber aufzulösen beginnen und danach völlig gelöst mit dem Speichel geschluckt werden; 2 bis 12 Gewichtsprozent Fett-bzw. Wachskomponenten; und 5 bis 40 Gewichtsprozent Füllstoffe, herstellbar durch ein Verfahren, bei welchem pulvrige Kaukomponenten mit dem geschmolzenen Fett-bzw. Wachskomponenten gemischt werden, worauf die Mischung anschließend unter Zusatz wenigstens einer Sirupkomponente in der Form einer konzentrierten Lösung eines Kohlenhydrats, Zuckeralkohols, oder einer konzentrierten Lösung von Gelatine oder gequollener Gelatine, oder einer konzentrierten Maltodextrinlösung zu einer krümeligen Masse wird, die man nun auf unter 0° C, vorzugweise auf unter -10° C, kühlt, anschließend auf höchstens 5 mm Korngröße vermahlt, und nach Kühlen auf unter 10° C, vorzugweise auf unter 0°C, zu Tabletten verpresst, die in bekannter Weise dragiert werden."

2. Kautablette nach Anspruch 1, **gekennzeichnet durch** folgende Zusammensetzung:
20 bis 50 Gewichtsprozent Kaukomponenten;
25 bis 45 Gewichtsprozent Sirupkomponenten;
4 bis 8 Gewichtsprozent Fett- bzw. Wachskomponenten;
15 bis 30 Gewichtsprozent Füllstoffe.

3. Kautablette nach Anspruch 1 oder 2 herstellbar durch ein Verfahren, bei welchem pulvrige Kaukomponenten mit den geschmolzenen Fett- bzw. Wachskomponenten gemischt werden, worauf die Mischung anschliessend unter Zusatz wenigstens einer konzentrierten Lösung der Sirupkomponente zu einer krümeligen Masse wird, die man nun auf unter 0°C, vorzugsweise auf unter -10°C, kühlt, anschliessend auf höchstens 5 mm Korngrösse vermahlt und nach Kühlen auf unter 10°C, vorzugsweise auf unter 0°C, zu Tabletten verpresst, die in bekannter Weise dragiert werden.

4. Kautablette nach einem der vorhergehenden Ansprüche, **dadurch gekennzeichnet, dass** sie ausserdem 0,1 bis 3, vorzugsweise 0,2 bis 0,5 Gewichtsprozent Glycerin oder Propylenglykol enthält.

5. Kautablette nach einem der vorhergehenden Ansprüche, **dadurch gekennzeichnet, dass** sie als Kaukomponente wenigstens eine der folgenden Substanzen enthält: (vorzugsweise sprühgetrocknetes) Gummi arabicum, Tragacanth, Guargum, Trockensirup - insbesondere Trocken-Glucose- und/oder Trocken-Fructosesirup, lösliches Cellulosederivat - insbesondere Natriumcarboxymethylcellulose.

6. Kautablette nach einem der vorhergehenden Ansprüche, **dadurch gekennzeichnet, dass** als Sirupkomponente wenigstens eine der folgenden Substanzen verwendet wird: Kohlehydratsirup - insbesondere etwa 80%iger Glucosesirup, oder Fructose-, Invertzucker-, Saccharose- und/oder Maissirup; konzentrierte - vorzugsweise etwa 70%ige - Maltodextrinlösung; Zuckeralkoholsirup, insbesondere von Sorbit, wie z.B. Karion® flüssig.

7. Kautablette nach einem der vorhergehenden Ansprüche, **dadurch gekennzeichnet, dass** als Fettkomponenten Triglyceride bzw. Glycerinester höherer Fettsäuren, insbesondere pflanzlichen oder tierischen Ursprungs, vorzugsweise der Grössenordnung C₁₀ bis C₂₂, deren Schmelzpunkt nicht über 60°C liegt, und als Wachskomponenten solche mit einem Schmelzpunkt von unter 70°C, insbesondere Bienenwachs und/oder Paraffin und/oder Ozokerit, verwendet werden.

8. Kautablette nach einem der vorhergehenden Ansprüche, **dadurch gekennzeichnet, dass** die Fettkomponenten bzw. die Mischung der Fett- und Wachskomponenten bei über 34°C, vorzugsweise bei etwa 45°C schmelzen.

9. Kautablette nach einem der vorhergehenden Ansprüche, **dadurch gekennzeichnet, dass** als Füllstoff wenigstens eine der folgenden Substanzen verwendet wird: pulvrige Kohlehydrate, insbesondere hydrolisierte Stärke, wie z.B. Maltodextrin; Zucker - insbesondere Fructose, Glucose und/oder Maltose; Zuckeralkohole - insbesondere Mannit, Sorbit und/oder Xylit.

10. Kautablette nach einem der vorhergehenden Ansprüche, **dadurch gekennzeichnet, dass** sie 0,1 bis 30 Gewichtsprozent eines - vorzugsweise in eine Matrix eingebetteten - pharmazeutischen Wirkstoffes, insbesondere einen Wirkstoff aus der folgenden Gruppe enthält: Antiemetika, wie z.B. Dimenhydrinat; Vitamin E; Herz-Kreislaufmittel, wie z.B.Salbutamol; Schleim- und Hustenlöser, wie z.B. Clobutinol; Antihistaminika, wie z.B. Cetirizin oder Loratadin; Mund- und Rachentherapeutika, wie z.B. Cetylpyridiniumchlorid, Benzalkoniumchlorid und Dequaliniumchlorid.

11. Kautablette nach Anspruch 9, **dadurch gekennzeichnet, dass** sie 0,5 bis 5, vorzugsweise 1 bis 4 Gew.% Dimenhydrinat; 30 bis 45, vorzugsweise 34 bis 37 Gew.% Glucosesirup; 20 bis 30 - vorzugsweise etwa 25 Gew.% Trockengklucosesirup; 4 bis 8, vorzugsweise 5 bis 7 Gew.% gehärtetes Kokosfett; 0.5 bis 2.0, vorzugsweise etwa 1 Gew.% Bienenwachs; 1 bis 8, vorzugsweise 2 bis 6 Gew.% Reisstärke; 12 bis 25, vorzugsweise 15 bis 20 Gew.% Gummi arabicum; und 4 bis 10, vorzugsweise 5 bis 8 Gew.% Maltodextrin enthält.

12. Kautablette nach Anspruch 10, **dadurch gekennzeichnet, dass** 1 Gewichtsteil Dimenhydrinat in 0,5 bis 2 Gewichtsteilen einer geschmacksmaskierenden Matrix aus 25 bis 40, vorzugsweise 30 bis 35 Gew.% wenigstens eines Polymetacrylsäureesters; und 3 bis 20, vorzugsweise 5 bis 15 Gew.% wenigstens eines Alkali- und/oder Erdalkalicarbonates bzw. -bikarbonates eingebettet ist.

## Claims

1. Soluble, gum-containing, coated chewable tablet containing 20 to 50 percent by weight of at least one syrup component; 10 to 60 percent by weight of chewable components which behave like chewing gum for at least 1 minute on chewing but begin to dissolve during this time and are then swallowed in completely dissolved form with the saliva; 2 to 12 percent by weight of fat or wax components; and 5 to 40 percent by weight of fillers, which can be prepared by a process in which pulverulent chewable components are mixed with the molten fat or wax components, whereupon, with addition of at least one syrup component in the form of a concentrated solution of a carbohydrate or of a sugar alcohol or of a concentrated solution of gelatin or swollen gelatin or of a concentrated maltodextrin solution, the mixture then becomes a crumbly mass, which is then cooled to below 0°C, preferably to below -10°C, then milled to a particle size of not more than 5 mm and, after cooling to below 10°C, preferably to below 0°C, compressed to give tablets, which are coated in a known manner.

2. Chewable tablet according to Claim 1, **characterized by** the following composition:
20 to 50 percent by weight of chewable components;
25 to 45 percent by weight of syrup components;
4 to 8 percent by weight of fat or wax components;
15 to 30 percent by weight of fillers.

3. Chewable component according to Claim 1 or 2, which can be prepared by a process in which pulverulent chewable components are mixed with the molten fat or wax components, whereupon, with addition of at least one concentrated solution of the syrup component, the mixture then becomes a crumbly mass, which is then cooled to below 0°C, preferably to below -10°C, then milled to a particle size of not more than 5 mm and, after cooling to below 10°C, preferably to below 0°C, compressed to give tablets, which are coated in a known manner.

4. Chewable tablet according to any of the preceding Claims, **characterized in that** it also contains 0.1 to 3, preferably 0.2 to 0.5, percent by weight of glycerol or propylene glycol.

5. Chewable table according to any of the preceding Claims, **characterized in that** it contains, as a chewable component, at least one of the following substances: (preferably spray-dried) gum arabic, tragacanth, guar gum, syrup in powder form - in particular glucose syrup in powder form and/or fructose syrup in powder form, soluble cellulose derivative - in particular sodium carboxymethylcellulose.

6. Chewable tablet according to any of the preceding Claims, **characterized in that** at least one of the following substances is used as the syrup component: carbohydrate syrup - in particular about 80% glucose syrup, or fructose, invert sugar, sucrose and/or maize syrup; concentrated - preferably about 70% - maltodextrin solution; sugar alcohol syrup, in particular of sorbitol, such as, for example, Karion® liquid.

7. Chewable tablet according to any of the preceding Claims, **characterized in that** triglycerides or glyceryl esters of higher fatty acids, in particular of vegetable or animal origin, preferably of the order of magnitude C₁₀ to C₂₂, whose melting point is not above 60°C, are used as fat components, and those having a melting point of less than 70°C, in particular beeswax and/or paraffin and/or ozokerite, are used as wax components.

8. Chewable tablet according to any of the preceding Claims, **characterized in that** the fat components or the mixture of the fat and wax components melt or melts at above 34°C, preferably at about 45°C.

9. Chewable tablet according to any of the preceding Claims, **characterized in that** at least one of the following substances is used as a filler: pulverulent carbohydrates, in particular hydrolyzed starch, such as, for example, maltodextrin; sugar - in particular fructose, glucose and/or maltose; sugar alcohols - in particular mannitol, sorbitol and/or xylitol.

10. Chewable tablet according to any of the preceding Claims, **characterized in that** it contains 0.1 to 30 percent by weight of a pharmaceutical active substance, preferably embedded in a matrix, in particular an active substance from the following group: antiemetics, such as, for example, dimenhydrinate; vitamin E; cardiovascular agents, such as, for example, salbutamol; expectorants, such as, for example, clobutinol; antihistamines, such as, for example, cetirizine or loratadine; therapeutic oropharyngeal agents, such as, for example, cetylpyridinium chloride, benzalkonium chloride and dequalinium chloride.

11. Chewable tablet according to Claim 9, **characterized in that** it contains 0.5 to 5, preferably 1 to 4, % by weight of dimenhydrinate; 30 to 45, preferably 34 to 37, % by weight of glucose syrup; 20 to 30, preferably about 25, % percent by weight of glucose syrup in powder form; 4 to 8, preferably 5 to 7, % by weight of hydrogenated coconut oil; 0.5 to 2.0, preferably about 1, % by weight of beeswax; 1 to 8, preferably 2 to 6, % by weight of rice starch; 12 to 25, preferably 15 to 20, % by weight of gum arabic; and 4 to 10, preferably 5 to 8, % by weight of maltodextrin.

12. Chewable tablet according to Claim 10, **characterized in that** 1 part by weight of dimenhydrinate is embedded in 0.5 to 2 parts by weight of a taste-masking matrix of 25 to 40, preferably 30 to 35, % by weight of at least one polymethacrylic ester; and 3 to 20, preferably 5 to 15, % by weight of at least one alkali metal and/or alkaline earth metal carbonate or bicarbonate.

## Revendications

1. Comprimé à mâcher soluble, dragéifié, contenant une gomme à mâcher et renfermant de 20 à 50 pour-cent en poids d'au moins un composant de type sirop ; de 10 à 60 pour-cent en poids de composant de type gomme à mâcher, qui, lorsque le comprimé est mâché, se comporte comme une gomme à mâcher pendant au moins 1 minute, tout en commençant à se dissoudre durant ce temps pour se dissoudre ensuite complètement et être avalé avec la salive ; de 2 à 12 pour-cent en poids de composant de type matière grasse ou, le cas échéant, de type cire ; et de 5 à 40 pour-cent en poids d'excipient ; obtenu par un procédé, dans lequel le composant de type gomme à mâcher en poudre est mélangé avec le composant de type matière grasse fondue ou, le cas échéant, de type cire fondue ; ensuite, au moins un composant de type sirop est ajouté sous la forme d'une solution concentrée d'un hydrate de carbone, d'un sucre-alcool ou d'une solution concentrée de gélatine ou de gélatine gonflée ou encore d'une solution concentrée de maltodextrine pour former une masse friable qui est alors refroidie à une température inférieure à 0 °C et, de préférence, inférieure à - 10 °C, puis broyée pour avoir une granulométrie de 5 mm au maximum et enfin, après refroidissement à moins de 10 °C et, de préférence, à moins de 0 °C, pressée en comprimés, qui sont dragéifiés par une méthode connue.

2. Comprimé à mâcher selon la revendication 1, **caractérisé par** la composition suivante :
20 à 50 pour-cent en poids de composant de type gomme à mâcher ;
25 à 45 pour-cent en poids de composant de type sirop ;
4 à 8 pour-cent en poids de composant de type matière grasse ou, le cas échéant, dé type cire ;
15 à 30 pour-cent en poids d'un excipient.

3. Comprimé à mâcher selon la revendication 1 ou la revendication 2, susceptible d'être obtenu par un procédé dans lequel le composant de type gomme à mâcher en poudre est mélangé avec le composant de type matière grasse fondue ou, le cas échéant, de type cire fondue, le mélange est ensuite additionné d'au moins une solution concentrée d'un composant de type sirop pour former une masse friable, qui est alors refroidie à moins de 0 °C et, de préférence, à moins de - 10 °C , puis broyée pour avoir une granulométrie de 5 mm au maximum et ensuite refroidie à moins de 10 °C et, de préférence, à moins de 0 °C, et pressée en comprimés qui sont dragéifiés par une méthode connue.

4. Comprimé à mâcher selon l'une des revendications précédentes, **caractérisé en ce qu'**il contient, en outre, de 0,1 à 3 et, de préférence, de 0,2 à 0,5 pour-cent en poids de glycérine ou de propylène glycol.

5. Comprimé à mâcher selon l'une des revendications précédentes, **caractérisé en ce que** le composant de type gomme à mâcher contient au moins une des substances suivantes (de préférence, séchée par atomisation) : gomme arabique, gomme adragante, gomme guar, matières sèches de sirop, en particulier des matières sèches d'un sirop de glucose et / ou de fructose et un dérivé soluble de la cellulose, en particulier la carboxyméthylcellulose sodique.

6. Comprimé à mâcher selon l'une des revendications précédentes, **caractérisé en ce que**, comme composant de type sirop, on utilise au moins une des substances suivantes : sirop d'un hydrate de carbone et, en particulier, un sirop à 80 % de glucose, de fructose, de sucre inverti ou de saccharose et / ou un sirop de maïs ; une solution concentrée, de préférence à 70 % environ, de maltodextrine ; un sirop d'un sucre-alcool, en particulier de sorbitol, comme par exemple le produit liquide Karion ®.

7. Comprimé à mâcher selon l'une des revendications précédentes, **caractérisé en ce que** l'on utilise en tant que composant de type matière grasse un triglycéride ou, le cas échéant, un ester de la glycérine et d'acides gras supérieurs, en particulier d'origine végétale ou animale, ayant, de préférence, une longueur de chaîne de l'ordre de C₁₀ à C₂₂ et un point de fusion ne dépassant pas 60 °C ; et, en tant que composant de type cire, un composant ayant un point de fusion en-dessous de 70 °C et, en particulier, une cire d'abeille et / ou une paraffine et / ou une ozokérite.

8. Comprimé à mâcher selon l'une des revendications précédentes, **caractérisé en ce que** le composant de type matière grasse ou, le cas échéant, le mélange de composants de type matière grasse et de type cire fond au-dessus de 34 °C et, de préférence, à 45 °C environ.

9. Comprimé à mâcher selon l'une des revendications précédentes, **caractérisé en ce que** l'on utilise, en tant qu'excipient, au moins une des substances suivantes : hydrate de carbone en poudre et, en particulier, un amidon hydrolysé comme par exemple une maltodextrine ; un sucre et, en particulier, le fructose, le glucose et / ou le maltose ; un sucre-alcool et, en particulier, le mannitol, le sorbitol et / ou le xylitol.

10. Comprimé à mâcher selon l'une des revendications précédentes, **caractérisé en ce qu'**il contient de 0,1 à 30 pour-cent en poids d'un agent pharmaceutique incorporé, de préférence, à une matrice et, en particulier, d'un agent provenant du groupe suivant : un antiémétique comme par exemple le dimenhydrinate ; la vitamine E ; un régulateur de la circulation cardiaque comme par exemple le salbutamol ; un mucolytique comme par exemple le clobutinol ; un antihistaminique comme par exemple la cétirizine ou la loratadine ; un médicament pour la bouche et pour la gorge comme par exemple le chlorure de cétylpyridinium, le chlorure de benzalkonium et le chlorure de déqualinium.

11. Comprimé à mâcher selon la revendication 9, **caractérisé en ce qu'**il contient de 0,5 à 5 et, de préférence, de 1 à 4 % en poids de dimenhydrinate ; de 30 à 45 et, de préférence, de 34 à 37 % en poids de sirop de glucose ; de 20 à 30 et, de préférence, environ 25 % en poids de matières sèches de sirop de glucose ; de 4 à 8 et, de préférence, de 5 à 7 % en poids de matières grasses durcies de coprah ; de 0,5 à 2,0 et, de préférence, environ 1 % en poids de cire d'abeille ; de 1 à 8 et, de préférence, de 2 à 6 % en poids d'amidon de riz ; de 12 à 25 et, de préférence, de 15 à 20 % en poids de gomme arabique ; et de 4 à 10 et, de préférence, de 5 à 8 % en poids de maltodextrine.

12. Comprimé à mâcher selon la revendication 10, **caractérisé en ce que** 1 partie en poids de dimenhydrinate est incorporée dans 0,5 à 2 parties en poids d'une matrice pour masquer le goût, constituée de 25 à 40 et, de préférence, de 30 à 35 % en poids d'au moins un ester de l'acide polyméthacrylique ; et de 3 à 20 et, de préférence, de 5 à 15 % en poids d'au moins un carbonate ou un bicarbonate d'un métal alcalin et / ou alcalino-terreux.
